# EUROPEAN PATENT APPLICATION

(11) **EP 3 808 858 A1**
(43) Date of publication of application: **21.04.2021**
(21) Application number: 19819116.5
(22) Date of filing: 13.06.2019
(51) Int. Cl.: C12Q 1/6883, G01N 33/68

(54) **BIOMARKER FOR PREDICTING EFFECTIVENESS FOR AUTOIMMUNE DISEASE TREATMENT, DIAGNOSTIC KIT, AND USE FOR TREATMENT**

(30) Priority: 15.06.2018 KR 20180069263
(71) Applicant: Corestem Co., Ltd., Cheongju-si, Chungcheongbuk-do 28420 (KR); Chungbuk National University Industry-Academy Cooperation Foundation, Cheongju-si, Chungcheongbuk-do 28644 (KR)
(72) Inventor: KIM, Kyung Suk, Seoul 02838 (KR); HAN, Sang Bae, Cheongju-si, Chungcheongbuk-do 28692 (KR)
(74) Representative: J A Kemp LLP
(86) International application number: PCT/KR2019/007121
(87) International publication number: WO 2019/240507

(57) **Abstract**

The present invention provides a biomarker for predicting effectiveness for autoimmune disease treatment, a diagnostic kit, and a use for treatment. More particularly, the present invention provides a biomarker for predicting the effectiveness of mesenchymal stem cells (MSC) comprising the chemokine receptor CXCR3 for autoimmune disease treatment; a biomarker comprising the chemokine CXCL10 for predicting the prognosis of autoimmune disease treatment; a diagnostic kit comprising an agent capable of measuring the expression of the biomarker; and a pharmaceutical composition for prevention or treatment of an autoimmune disease, which comprises mesenchymal stem cells having an upregulated expression level of the chemokine receptor CXCR3.

<110>    CoreStem CO.,LTD.,  Chungbuk National University Industry-Academy
Cooperation Foundation

<120>    BIOMARKER FOR TREATMENT OF AUTOIMMUNE DISEASES, DIAGNOSTIC KIT
         AND THE THERAPEUTIC USE THEREOF

<130>    PCT5040224

<150>    KR 10-2018-0069263
<151>    2018-06-15

<160>    32

<170>    KoPatentIn 3.0

<210>    1
<211>    20
<212>    DNA
<213>    Artificial Sequence

<220>
<223>    sence primer for CCR5


<400>    1
gctgaagagc gtgactgata                                                   20


<210>    2
<211>    20
<212>    DNA
<213>    Artificial Sequence

<220>
<223>    antisence primer for CCR5


<400>    2
gaggactgca tgtataatga                                                   20


<210>    3
<211>    25
<212>    DNA
<213>    Artificial Sequence

<220>
<223>    sence primer for CCR7


<400>    3
acagcggcct ccagaagaac agcgg                                             25


<210>    4
<211>    25
<212>    DNA
<213>    Artificial Sequence

<220>
<223>    antisence primer for CCR7

<400>    4
tgacgtcata ggcaatgttg agctg                                          25


<210>    5
<211>    19
<212>    DNA
<213>    Artificial Sequence

<220>
<223>    sence primer for CXCR3


<400>    5
gtacacgcag agcagtgcg                                                 19


<210>    6
<211>    24
<212>    DNA
<213>    Artificial Sequence

<220>
<223>    antisence primer for CXCR3


<400>    6
gaacgtcaag tgctagatgc ctcg                                           24


<210>    7
<211>    20
<212>    DNA
<213>    Artificial Sequence

<220>
<223>    sence primer for CXCR4


<400>    7
ggctgtagag cgagtgttgc                                                20


<210>    8
<211>    21
<212>    DNA
<213>    Artificial Sequence

<220>
<223>    antisence primer for CXCR4


<400>    8
gtagaggttg acagtgtaga t                                              21


<210>    9
<211>    24
<212>    DNA
<213>    Artificial Sequence

<220>
<223>    sence primer for iNOS

<400>    9
ccttccgaag tttctggcag cagc                                              24


<210>    10
<211>    25
<212>    DNA
<213>    Artificial Sequence

<220>
<223>    antisence primer for iNOS


<400>    10
ggctgtcaga gcctcgggct ttggg                                             25


<210>    11
<211>    20
<212>    DNA
<213>    Artificial Sequence

<220>
<223>    sence primer for TGF-beta


<400>    11
tgacgtcact ggagttgtac                                                   20


<210>    12
<211>    20
<212>    DNA
<213>    Artificial Sequence

<220>
<223>    antisence primer for TGF-beta


<400>    12
ggttcatgtc atggatggtg                                                   20


<210>    13
<211>    20
<212>    DNA
<213>    Artificial Sequence

<220>
<223>    sence primer for COX-2


<400>    13
ggagagacta tcaagatagt                                                   20


<210>    14
<211>    20
<212>    DNA
<213>    Artificial Sequence

<220>
<223>    antisence primer for COX-2


<400>    14
atggtcagta gacttttaca                                                    20


<210>    15
<211>    25
<212>    DNA
<213>    Artificial Sequence

<220>
<223>    sence primer for beta-actin


<400>    15
tggaatcctg tggcatccat gaaac                                              25


<210>    16
<211>    25
<212>    DNA
<213>    Artificial Sequence

<220>
<223>    antisence primer for beta-actin


<400>    16
taaaacgcag ctcagtaaca gtccg                                              25


<210>    17
<211>    20
<212>    DNA
<213>    Artificial Sequence

<220>
<223>    sence primer for CXCL10


<400>    17
ggctggtcac ctttcagaag                                                    20


<210>    18
<211>    20
<212>    DNA
<213>    Artificial Sequence

<220>
<223>    antisence primer for CXCL10


<400>    18
atggatggac agcagagagc                                                    20


<210>    19
<211>    20
<212>    DNA

<213>    Artificial Sequence

<220>
<223>    sence primer for CXCL12


<400>    19
gagccaacgt caagcatctg                                                    20


<210>    20
<211>    20
<212>    DNA
<213>    Artificial Sequence

<220>
<223>    antisence primer for CXCL12


<400>    20
cgggtcaatg cacacttgtc                                                    20


<210>    21
<211>    19
<212>    DNA
<213>    Artificial Sequence

<220>
<223>    sence primer for MMP-2


<400>    21
caagttcccc ggcgatgtc                                                     19


<210>    22
<211>    21
<212>    DNA
<213>    Artificial Sequence

<220>
<223>    antisence primer for MMP-2


<400>    22
ttctggtcaa ggtcacctgt c                                                  21


<210>    23
<211>    21
<212>    DNA
<213>    Artificial Sequence

<220>
<223>    sence primer for MMP-9


<400>    23
ctggacagcc agacactaaa g                                                  21


<210>    24

<211>    20
<212>    DNA
<213>    Artificial Sequence

<220>
<223>    antisence primer for MMP-9


<400>    24
ctcgcggcaa gtcttcagag                                                    20


<210>    25
<211>    20
<212>    DNA
<213>    Artificial Sequence

<220>
<223>    sence primer for TIMP-1


<400>    25
ttatcccatt ggggcattta                                                    20


<210>    26
<211>    20
<212>    DNA
<213>    Artificial Sequence

<220>
<223>    antisence primer for TIMP-1


<400>    26
ttgctgcctt tgactgattg                                                    20


<210>    27
<211>    26
<212>    DNA
<213>    Artificial Sequence

<220>
<223>    sence primer for Integrin alpha 4


<400>    27
ctccctcaag atgataagtt gttcaa                                             26


<210>    28
<211>    24
<212>    DNA
<213>    Artificial Sequence

<220>
<223>    antisence primer for Integrin alpha 4


<400>    28
tgtgcaaatg tacactctct tcca                                               24

<210>	29
<211>	18
<212>	DNA
<213>	Artificial Sequence

<220>
<223>	sence primer for Integrin beta 1


<400>	29
ggctgaagat taccctat	18


<210>	30
<211>	18
<212>	DNA
<213>	Artificial Sequence

<220>
<223>	antisence primer for Integrin beta 1


<400>	30
cattcatcaa atccgttc	18


<210>	31
<211>	23
<212>	DNA
<213>	Artificial Sequence

<220>
<223>	sence primer for CD44


<400>	31
gaccggttac cataactatt gtc	23


<210>	32
<211>	21
<212>	DNA
<213>	Artificial Sequence

<220>
<223>	antisence primer for CD44


<400>	32
catcgatgtc ttcttggtgt g	21

## Description

### [Technical Field]

The present application claims priority to and the benefit of Korean Patent Application No. 10-2018-0069263 filed in the Korean Intellectual Property Office on June 15, 2018, the entire contents of which are incorporated herein by reference.

The present invention relates to a biomarker for predicting effectiveness for autoimmune disease treatment, a diagnostic kit, and a use for treatment.

### [Background Art]

Systemic lupus erythematosus (SLE) is a systemic autoimmune disease with multiple dysfunctions, and is characterized by the deposition of immune complexes and the invasion of inflammatory cells.

Lupus nephritis occurs in 60% of patients with SLE, and is a major cause of increased morbidity and mortality. Since mesenchymal stem cells (MSCs) can suppress T cells, B cells, dendritic cells, and NK cells in soluble factor- and contact-dependent manners, and promote Treg cells, MSCs have been studied as a promising alternative therapy.

In order to exert efficacy *in vivo*, MSCs efficiently penetrate an inflammatory area. Chemokines are produced in the nephritic kidney, and MSCs express chemokine receptors. CCL2, CCL3, CCL5, CXCL10, CXCL12, CXCL13, and CX3CL1 are expressed in the nephritic kidneys of lupus model animals and patients with SLE and increase renal infiltration of inflammatory cells. Further, bone marrow-derived MSCs express CCR2, CCR5, CXCR3, and CXCR4, but little is known about how MSCs penetrate an inflammatory tissue with respect to chemokine reactivity. Several papers have shown that MSCs penetrate inflammatory tissues using the CXCL12-CXCR4 axis. However, there has been no report on the role of the CXCL10-CXCR3 axis in MSC infiltration into nephritic kidneys.

### Prior Art Documents

Yang D, Sun S, Wang Z, Zhu P, Yang Z and Zhang B. Stromal cell-derived factor-1 receptor CXCR4-overexpressing bone marrow mesenchymal stem cells accelerate wound healing by migrating into skin injury areas. Cellular Reprogramming. 2013; 15: 206-15

### [Disclosure]

### [Technical Problem]

The present invention provides a biomarker for predicting effectiveness for autoimmune disease treatment, a diagnostic kit, and a use for treatment. More particularly, the present invention provides a biomarker for predicting the effectiveness of mesenchymal stem cells (MSC) comprising the chemokine receptor CXCR3 for autoimmune disease treatment; a biomarker comprising the chemokine CXCL10 for predicting the prognosis of autoimmune disease treatment; a diagnostic kit comprising an agent capable of measuring the expression of the biomarker; and a pharmaceutical composition for prevention or treatment of an autoimmune disease, which comprises mesenchymal stem cells having an upregulated expression level of the chemokine receptor CXCR3.

### [Technical Solution]

The present invention provides a biomarker composition for predicting effectiveness of mesenchymal stem cells comprising the chemokine receptor CXCR3 for autoimmune disease treatment.

The present invention also provides a kit comprising an agent which measures the expression level of CXCR3 or the expression level of mRNA thereof for predicting effectiveness of mesenchymal stem cells for autoimmune disease treatment.

In an exemplary embodiment, an agent which measures the expression level of CXCR3 is an antibody specifically binding to CXCR3, and an agent which measures the expression level of mRNA of CXCR3 may be a primer, probe or antisense nucleotide specifically binding to CXCR3. Further, the kit may be an RT-PCR kit, a competitive RP-PCR kit, a real-time RT-PCR kit, a quantitative RT-PCR kit, or a DNA chip kit.

It is obvious that the antibody, primer, probe or antisense nucleotide can be made using techniques known in the art to which the present invention pertains.

In the kit, when CXCR3 or mRNA thereof is not expressed, or is expressed at a level lower than that of wild-type mesenchymal stem cells, it can be predicted that the therapeutic effectiveness is absent or low. As described above, by measuring the expression of the biomarker CXCR3 or mRNA thereof, effectiveness of mesenchymal stem cells for autoimmune disease treatment can be predicted.

The autoimmune disease may be selected from the group consisting of lupus (systemic lupus erythematosus), rheumatoid arthritis, progressive systemic sclerosis (scleroderma), atopic dermatitis, alopecia areata, psoriasis, pemphigus, asthma, aphthous stomatitis, chronic thyroiditis, inflammatory enteritis, Behcet's disease, Crohn's disease, dermatomyositis, polymyositis, multiple sclerosis, autoimmune hemolytic anemia, autoimmune encephalomyelitis, myasthenia gravis, Grave's disease, polyarteritis nodosa, ankylosing spondylitis, fibromyalgia syndrome, and temporal arteritis, and may be particularly systemic lupus erythematosus or complications caused thereby.

The complications may include one or more selected from the group consisting of kidney damage, headaches, dizziness, behavior changes, vision problems, stroke, seizures, anemia, hemorrhage, blood clotting, vasculitis, pleurisy, pneumonia, pericarditis, cardiovascular diseases, heart attacks, arthritis, arthralgia, and myalgia, but is not limited thereto.

The present invention also provides a biomarker composition comprising the chemokine CXCL10 for predicting the prognosis of autoimmune disease treatment.

In an exemplary embodiment, the autoimmune disease treatment may be a treatment using mesenchymal stem cells comprising CXCR3. Further, CXCL10 is preferably expressed in the kidneys. In the kidneys expressing the CXCL10, CXCL10 can induce the infiltration of mesenchymal stem cells comprising CXCR3 into the kidneys.

The present invention also provides a kit comprising an agent which measures the expression level of CXCL10 for predicting the prognosis of autoimmune disease treatment.

In an exemplary embodiment, the agent which measures the expression level of CXCL10 may be an antibody specifically binding to CXCR3. It is obvious that the antibody can be made using techniques known in the art to which the present invention pertains.

In some exemplary embodiments of the present invention, it is described that only the expression level of CXCR3 and/or CXCL10 is measured, but it is obvious that the measurement of the expression level of each biomarker is included by measuring the expression of mRNA thereof.

The present invention also provides a method for providing information for predicting effectiveness of mesenchymal stem cells for autoimmune disease treatment by measuring the expression level of the chemokine receptor CXCR3 expressed in mesenchymal stem cells (MSCs) or the expression level of mRNA thereof.

A method for measuring the biomarker level includes reverse transcription-polymerase chain reaction (RT-PCR), competitive RT-PCR, real time quantitative RT-PCR, quantitative RT-PCR, an RNase protection method, northern blotting or DNA chip technology, immunohistochemical staining, immunoprecipitation assay, complement fixation assay, immunofluorescence, or the like.

In an exemplary embodiment, the autoimmune disease may be particularly systemic lupus erythematosus or complications caused thereby.

The present invention also provides a method for screening mesenchymal stem cells exhibiting an effective therapeutic effect on an autoimmune disease by measuring the expression level of the chemokine receptor CXCR3 or the expression level of mRNA thereof.

Mesenchymal stem cells selected by the above screening method have an excellent activity of preventing or treating an autoimmune disease. Therefore, it is possible to provide a pharmaceutical composition comprising the mesenchymal stem cells screened by the method for preventing or treating an autoimmune disease.

Further, the present invention may provide a method for preventing or treating an autoimmune disease, the method comprising administering or taking a pharmaceutical composition comprising the mesenchymal stem cells screened by the method to an individual.

In addition, the present invention may provide a use of a pharmaceutical composition comprising the mesenchymal stem cells screened by the method for preventing or treating an autoimmune disease.

The present invention also provides a pharmaceutical composition for preventing or treating an autoimmune disease, which comprises: 1) CXCR3 itself, which is a chemokine receptor of mesenchymal stem cells; or 2) an agent which increases the expression level of CXCR3.

Furthermore, the present invention may provide a method for preventing or treating an autoimmune disease, the method comprising administering, to an individual, or the individual taking, a pharmaceutical composition comprising: a chemokine receptor CXCR3 of mesenchymal stem cells; or an agent which increases the expression level of CXCR3.

Further, the present invention may provide a use of a pharmaceutical composition comprising: a chemokine receptor CXCR3 of mesenchymal stem cells; or an agent which increases the expression level of CXCR3 for preventing or treating an autoimmune disease.

In an exemplary embodiment, the agent which increases the expression level of CXCR3 may be an antibody specifically binding to CXCR3. In the case of the antibody, it should be determined that derivatives thereof or biological equivalents thereof are included, and it is obvious that the antibody, derivatives thereof or biological equivalents thereof can be made using techniques known in the art to which the present invention pertains. Derivatives of the antibody or biological equivalents thereof refer to those exhibiting the equivalent activity of an antibody specifically binding to CXCR3 in order to increase the expression level of CXCR3, and include mutations caused by changes in amino acid sequences and deletions, attachments, and the like of amino acid sequences, or various substituents.

The present invention also provides a pharmaceutical composition for preventing or treating an autoimmune disease, comprising, as an active ingredient, mesenchymal stem cells having an upregulated expression level of the chemokine receptor CXCR3 compared to the wild type.

In addition, the present invention may provide a method for preventing or treating an autoimmune disease, the method comprising administering, to an individual, or the individual taking, a pharmaceutical composition comprising, as an active ingredient, mesenchymal stem cells having an upregulated expression level of the chemokine receptor CXCR3 compared to the wild type.

Furthermore, the present invention may provide a use of a pharmaceutical composition comprising, as an active ingredient, mesenchymal stem cells having an upregulated expression level of the chemokine receptor CXCR3 compared to the wild type for preventing or treating an autoimmune disease.

In an exemplary embodiment, the mesenchymal stem cells having an upregulated expression level of CXCR3 compared to the wild type have: (i) positive immunological properties for at least one surface antigen selected from the group consisting of CD105, CD29, CD44, CD73, and CD90; and (ii) negative immunological properties for at least one surface antigen selected from the group consisting of CD34, CD45, and HLA-DR.

The autoimmune disease may be selected from the group consisting of lupus (systemic lupus erythematosus), rheumatoid arthritis, progressive systemic sclerosis (scleroderma), atopic dermatitis, alopecia areata, psoriasis, pemphigus, asthma, aphthous stomatitis, chronic thyroiditis, inflammatory enteritis, Behcet's disease, Crohn's disease, dermatomyositis, polymyositis, multiple sclerosis, autoimmune hemolytic anemia, autoimmune encephalomyelitis, myasthenia gravis, Grave's disease, polyarteritis nodosa, ankylosing spondylitis, fibromyalgia syndrome, and temporal arteritis, and may be particularly systemic lupus erythematosus or complications caused thereby.

When the composition of the present invention is prepared as a pharmaceutical composition, the pharmaceutical composition of the present invention may include a pharmaceutically acceptable carrier. A pharmaceutically acceptable carrier included in the pharmaceutical composition of the present invention is typically used in formulation, and includes lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, saline, phosphate buffered saline (PBS) or a medium, and the like, but is not limited thereto.

The pharmaceutical composition of the present invention may be administered orally, topically (buccal, sublingual, dermal, or ocular), transdermally or parenterally, and may be administered preferably parenterally, and more preferably intravascularly.

A suitable dose of the pharmaceutical composition of the present invention may vary depending on factors, such as formulation method, administration method, age, body weight, sex or disease condition of the patient, diet, administration time, administration route, excretion rate and response sensitivity. A typical dose of the pharmaceutical composition of the present invention is 10² to 10¹⁰ cells per day on an adult basis.

The present invention also provides a use of mesenchymal stem cells having an upregulated expression level of the chemokine receptor CXCR3 compared to the wild type for preparing a pharmaceutical agent for preventing or treating an autoimmune disease.

The present invention also provides a method for treating an autoimmune disease, the method comprising: 1) measuring the expression level of the chemokine receptor CXCR3 or mRNA thereof expressed in mesenchymal stem cells (MSCs); and
2) administering mesenchymal stem cells having an upregulated expression level of the chemokine receptor CXCR3 compared to the wild type to an individual in need of treatment of an autoimmune disease.

In an exemplary embodiment, the mesenchymal stem cells having an upregulated expression level of CXCR3 compared to the wild type have: (i) positive immunological properties for at least one surface antigen selected from the group consisting of CD105, CD29, CD44, CD73, and CD90; and (ii) negative immunological properties for at least one surface antigen selected from the group consisting of CD34, CD45, and HLA-DR.

The mesenchymal stem cells having (i) positive immunological properties for at least one surface antigen selected from the group consisting of CD105, CD29, CD44, CD73, and CD90; and (ii) negative immunological properties for at least one surface antigen selected from the group consisting of CD34, CD45, and HLA-DR are described in detail in Korean Patent Application No. 10-2014-0097129 of the present applicant CORESTEM, Inc, the contents of which are hereby incorporated by reference in their entirety.

### [Advantageous Effects]

According to the present invention, it is possible to provide a biomarker for predicting effectiveness for autoimmune disease treatment, a diagnostic kit, and a use for treatment. In particular, the present invention can provide a biomarker for predicting the effectiveness of mesenchymal stem cells (MSC) comprising the chemokine receptor CXCR3 for autoimmune disease treatment; a biomarker comprising the chemokine CXCL10 for predicting the prognosis of autoimmune disease treatment; a diagnostic kit comprising an agent capable of measuring the expression of the biomarker; and a pharmaceutical composition for prevention or treatment of an autoimmune disease, which comprises mesenchymal stem cells having an upregulated expression level of the chemokine receptor CXCR3. In particular, the present invention can clearly elucidate the role of the CXCL10-CXCR3 axis in MSC infiltration, and thus can show or improve a preventive or therapeutic effect on an autoimmune disease, particularly systemic lupus erythematosus.

### [Description of Drawings]

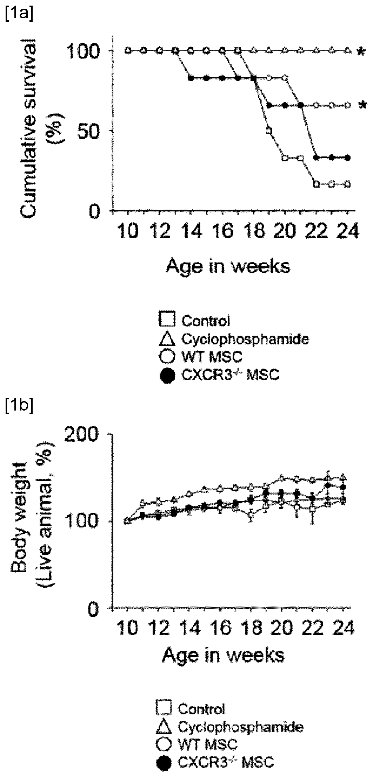

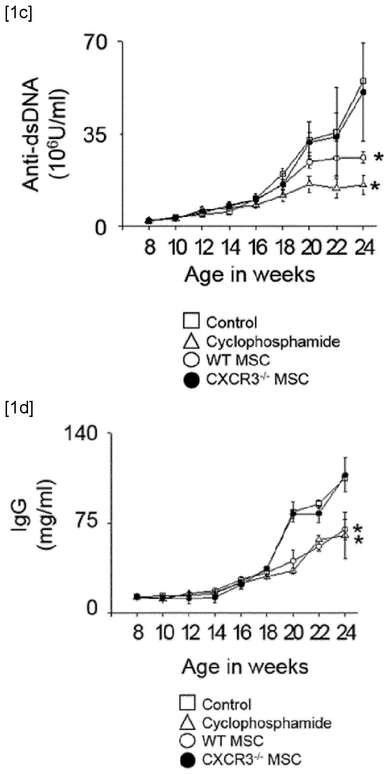

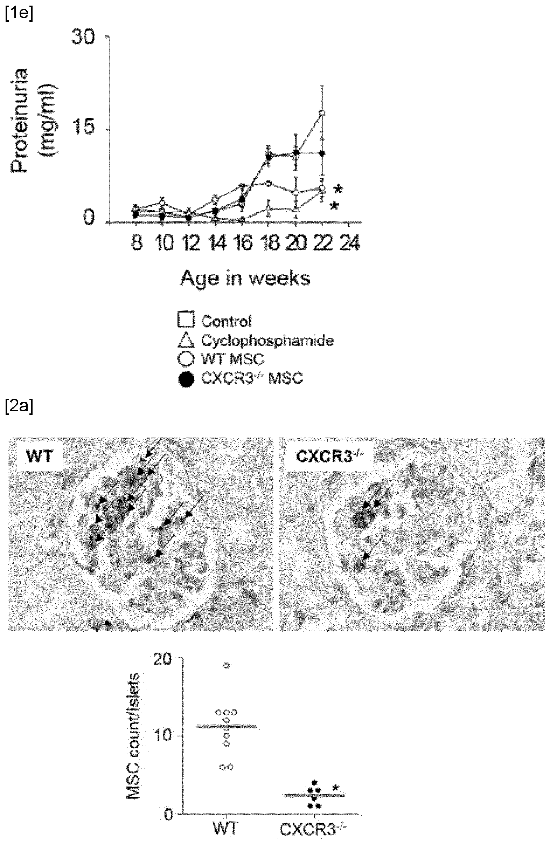

FIG. 1 is a graph showing the results of measuring the viability (FIG. 1A), body weight (FIG. 1B), anti-dsDNA IgG level (FIG. 1C), and total IgG level (FIG. ID) after intravenously injecting MSCs or cyclophosphamide (CP) once into 12-week-old MRL.Fas^{lpr} mice. WT: wild type, CXCR3 -/-: CXCR3 deficient type (*p < 0.01 versus control).

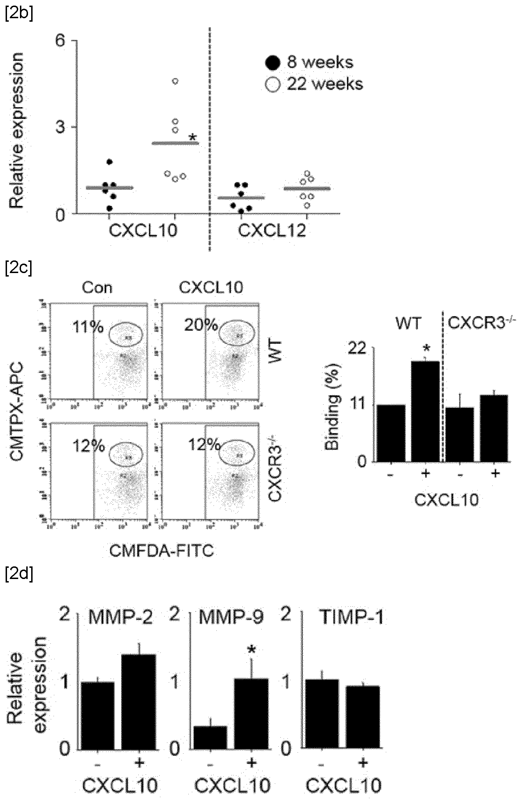

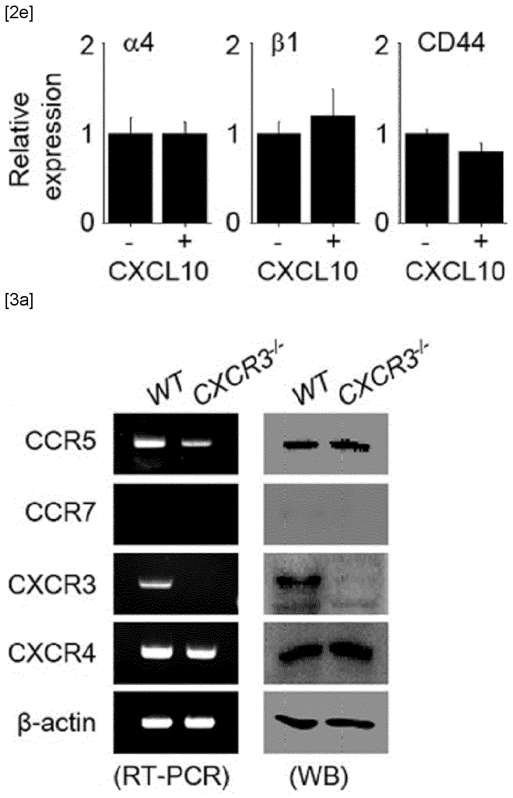

FIG. 2 is an analysis of renal infiltration of mesenchymal stem cells expressing CXCR3. FIG. 2 illustrates that MSCs (H-2^{d}) derived from C57BL/5 mice are intravenously injected into MRL.Fas^{lpr} mice (H-2^{k}), the spleens are isolated after 24 hours and stained using an anti-mouse MHC-I (H-2^{d})antibody, and then the number of H-2^{d} positive MSCs per islet is counted. FIG. 2B illustrates the results of real-time PCR measurement of gene expression levels of CXCL10 and CXCL12 after isolating total RNA from the kidneys of 8-week- or 22-week-old MRL.Fas^{lpr} mice. *p < 0.01 versus control. FIG. 2C illustrate the results of analyzing the binding rates of CMTMR-labeled MSCs and CMFDA-labeled endothelial cells using flow cytometry (n = 3). A conjugation ratio was calculated as a portion of the CMFDAC MTPX double-positive events. *p < 0.01. FIG. 2D illustrates the results of measuring gene expression levels of MMP-2, MMP-9 and TIMP-1 with real-time PCR after treating MSCs with 100 ng ml of CXCL10 for 24 hours. *p < 0.01. FIG. 2E illustrates the results of measuring the gene expression degrees of α4 integrin, β1 integrin and CD44 using real-time PCR after treating MSCs with 100 ng ml of CXCL10 for 24 hours. *p < 0.01.

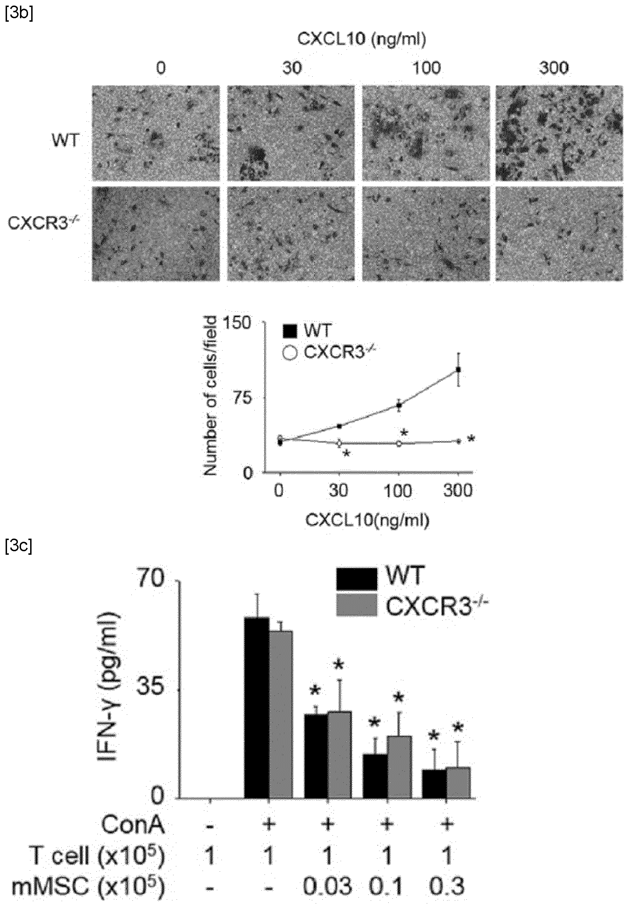

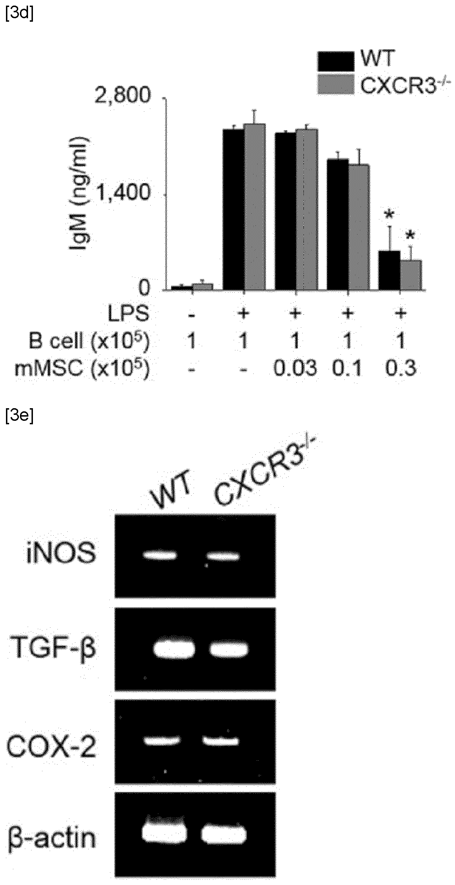

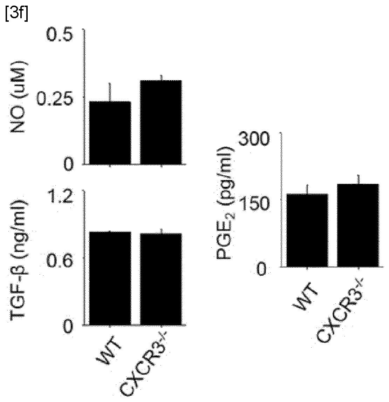

FIG. 3 illustrates the result of analyzing the immunosuppressive ability of CXCR3 -/- MSCs. FIG. 3A illustrates the results of isolating the total RNA and proteins from MSCs produced from WT and CXCR3 -/- mice and measuring the levels of CCR5, CCR7, CXCR3, and CXCR4 by real-time PCR (RT-PCR) and western blot (WB). FIG. 3B illustrates the results of analyzing MSC migration. The MSC migration was confirmed by a Transwell assay using Transwell plates with an 8 µm pore filter (Costar, Corning, Cambridge, MA, USA). After an upper well was coated with 0.1% gelatin (Sigma-Aldrich, St. Louis, MO, USA) at 37°C for 2 hours, the upper well was loaded with 2 × 10⁴ MSCs in 200 µl of a medium, 500 µl of a medium containing 10% FBS and 100 ng/ml CXCL10 (R & D Systems, Minneapolis, MN, USA) was added to a lower well, and then non-migratory cells at the upper portion of the filter were removed after 24 hours, and after a membrane was fixed in 10% formalin, MSCs migrated to the lower portion of the filter were stained with 0.5% Crystal Violet for 10 minutes, and the number of migrated MSCs was counted under an inverted light microscope. *p < 0.01. FIG 3C illustrates the results of adding MSCs at 0.03 to 0.3 × 10⁵ cells/well to a 96 well plate, adding T cells at 1 × 10⁵ cells/well thereto, and adding 1 µg/ml of concanavalin A thereto to culture the cells for 72 hours, and then measuring the level of IFN-γ accumulated in the medium using an ELISA kit. *p < 0.01. FIG. 3D illustrates the results of adding MSCs at 0.03 to 0.3 × 10⁵ cells/well to a 96 well plate, adding B cells at 1 × 10⁵ cells/well thereto, and adding 1 µg/ml of lipopolysaccharide thereto to culture the cells for 72 hours, and then measuring the level of IgM accumulated in the medium using an ELISA kit. *p < 0.01. FIGS. 3E and 3F illustrate the results of replacing the medium with a new medium on day 20 of the MSC culture and measuring the level of soluble factors in the medium after 24 hours. NO levels were measured with the Griess reagent and TGF-β and PGE2 levels were measured using an ELISA kit (R & D systems, Minneapolis, MN, USA).

### [Modes of the Invention]

Hereinafter, the present invention will be described in more detail through the Examples. The objects, features and advantages of the present invention will be readily understood through the following Examples. The present invention is not limited to the Examples described herein, and may be implemented in different forms. The examples introduced herein are provided such that the idea of the present invention can be sufficiently conveyed to a person with ordinary skill in the art to which the present invention pertains. Therefore, the present invention should not be limited by the following Examples.

### Examples

### Materials and methods

### Mesenchymal stem cells

Mouse MSCs were obtained from bone marrow cells of the tibiae and femurs of 6- to 8-week-old C57BL/6 mice (Orient Bio, Gyeonggi Province, Korea) or CXCR3 -/- (B6.129P2-Cxcr3^{tm1Dgen}/J) mice (Jackson Laboratory, Bar Harbor, ME, USA)(CXCR3 -/- represents a CXCR3 deficient form). BM cells were cultured in an α-MEM medium containing 10% fetal bovine serum (FBS), 2 mM L-glutamine, and penicillin/streptomycin under 37°C and 5% CO₂ conditions. Non-adherent cells were removed on day 1, and adherent cells were cultured by replenishing the medium every three days, and then used between day 17 and day 20. All animal experiments were performed according to the guidelines approved by the Chungbuk National University Institutional Animal Care and Use Committees.

### MRL.Fas^{lpr} mouse model

MRL.*Fas^{lpr}* mice were purchased from Jackson Laboratory (Bar Harbor, Maine, USA). The mice were stored under a 12 h light/dark cycle and in a state where there was no specific pathogen under 21 to 24°C and 40 to 60% relative humidity conditions. Phosphate buffered saline (PBS, control, n = 6), cyclophosphamide (50 mg/kg, n = 6), wild-type MSCs (1×10⁶ cells/inj ection, n = 6), and CXCR3 -/- MSCs (1 × 10⁶ cells/injection, n = 6) were intraperitoneally injected into MRL.*Fas*^{lpr} mice once in 12 weeks. The viability and body weight were measured every week. Protein levels in urine and anti-ds DNA IgG and total IgG in serum were measured using ELISA kits purchased from Sigma-Aldrich, Alpha Diagnostic International (San Antonio, TX, USA) and eBioscience (San Diego, CA, USA). For immunohistochemistry, MSCs (H-2^{d}) were intravenously injected at 1 × 10⁶ cells/injection into 18-week-old MRL_{.}Fas^{lpr} mice (H-2^{k}). After 24 hours, the number of MSCs infiltrated into the kidneys was determined using a primary mouse antibody against mouse MHC-1 (H-2^{d}) and a secondary antibody anti-mouse IgG conjugated with horseradish peroxidase (HRP; Vector Laboratories, Burlingame, CA, USA), and counter-staining was performed with hematoxylin.

### Real-time-PCR

Total RNA was isolated from spleen cells using TRIzol Reagent (Thermo Fisher Scientific). RNA was quantified using a spectrophotometer and stored at a concentration of 1 mg/ml at -80°C. cDNA was synthesized from 3 µg of total RNA using an RT kit (Bioneer, Daejeon, Korea). The expression levels of mRNA for CCR5, CCR7, CXCR3, CXCR4, iNOS, TGF-β, and COX-2 were analyzed by PCR. The sequences of primers used are as follows: CCR5, sense, 5'-GCT GAA GAG CGT GAC TGA TA-3' (SEQ ID NO: 1), antisense, 5'-GAG GAC TGC ATG TAT AAT GA-3' (SEQ ID NO: 2); CCR7, sense, 5'-ACA GCG GCC TCC AGA AGA ACA GCG G-3' (SEQ ID NO: 3), antisense, 5'-TGA CGT CAT AGG CAA TGT TGA GCT G-3' (SEQ ID NO: 4); CXCR3, sense, 5'-GTA CAC GCA GAG CAG TGC G-3' (SEQ ID NO: 5), antisense, 5'-GAA CGT CAA GTG CTA GAT GCC TCG-3' (SEQ ID NO: 6); CXCR4, sense, 5'-GGC TGT AGA GCG AGT GTT GC-3' (SEQ ID NO: 7), antisense, 5'-GTA GAG GTT GAC AGT GTA GAT-3' (SEQ ID NO: 8); iNOS, sense, 5'-CCT TCC GAA GTT TCT GGC AGC AGC-3' (SEQ ID NO: 9), antisense, 5'-GGC TGT CAG AGC CTC GGG CTT TGG G-3' (SEQ ID NO: 10); TGF-β, sense, 5'-TGA CGT CAC TGG AGT TGT AC-3' (SEQ ID NO: 11), antisense, 5'-GGT TCA TGT CAT GGA TGG TG-3' (SEQ ID NO: 12); COX-2, sense, 5'-GGA GAG ACT ATC AAG ATA GT-3' (SEQ ID NO: 13), antisense, 5'-ATG GTC AGT AGA CTT TTA CA-3' (SEQ ID NO: 14); β-actin, sense, 5'-TGG AAT CCT GTG GCA TCC ATG AAA C-3' (SEQ ID NO: 15), and antisense, 5'-TAA AAC GCA GCT CAG TAA CAG TCC G-3' (SEQ ID NO: 16) are included. APCR product was isolated in 1% agarose gel and stained with 5 µg/ml ethidium bromide.

Quantitative PCR was performed using SYBR Green Master Mix (Qiagen, Hilden, GER) and a StepOnePlus real-time PCR system (Applied Biosystems, CA, USA). The sequences of primers used are as follows: CXCL10, sense, 5'-GGC TGG TCA CCT TTC AGA AG-3' (SEQ ID NO: 17), antisense, 5'-ATG GAT GGA CAG CAG AGAGC-3' (SEQ ID NO: 18); CXCL12, sense, 5'-GAG CCA ACG TCA AGC ATC TG-3' (SEQ ID NO: 19), antisense, 5'-CGG GTC AAT GCA CAC TTG TC-3' (SEQ ID NO: 20); MMP-2, sense, 5'-CAA GTT CCC CGG CGA TGT C-3' (SEQ ID NO: 21), antisense, 5'-TTC TGG TCA AGG TCA CCT GTC-3' (SEQ ID NO: 22); MMP-9, sense, 5'-CTG GAC AGC CAG ACA CTA AAG-3' (SEQ ID NO: 23), antisense, 5'-CTC GCG GCA AGT CTT CAG AG-3' (SEQ ID NO: 24); TIMP-1, sense, 5'-TTA TCC CAT TGG GGC ATT TA-3' (SEQ ID NO: 25), antisense, 5'-TTG CTG CCT TTG ACT GAT TG-3' (SEQ ID NO: 26); integrin α4, sense, 5'-CTC CCT CAA GAT GAT AAG TTG TTC AA' (SEQ ID NO: 27), antisense, 5'-TGT GCA AAT GTA CAC TCT CTT CCA-3' (SEQ ID NO: 28); integrin β1, sense, 5'-GGC TGA AGA TTA CCC TAT' (SEQ ID NO: 29), antisense, 5'-CAT TCA TCA AAT CCG TTC-3' (SEQ ID NO: 30); CD44, sense, 5'-GAC CGG TTA CCA TAA CTA TTG TC' (SEQ ID NO: 31), antisense, 5'-CAT CGA TGT CTT CTT GGT GTG-3' (SEQ ID NO: 32). The relative mRNA amount of each sample was calculated based on the threshold cycle (Ct) thereof compared with the threshold cycle (Ct) of a housekeeping gene β-actin.

### Western blot

After cell lysates were prepared and detergent-insoluble materials were removed, the same amount of proteins were fractionated with 10% SDS-PAGE, and transferred to a pure nitrocellulose membrane. The membrane was blocked with TBS/Tween 20 (TTBS) containing 5% bovine serum albumin (BSA) for 1 hour and then incubated with a suitable dilution of the primary antibody in TTBS containing 5% BSA for 2 hours.

The blot was cultured with a biotinylated secondary antibody for 1 hour, and then incubated with HRP-conjugated streptavidin for 1 hour. Signals were detected by enhanced chemiluminescence (ECL; Amersham Pharmacia Biotech, Piscataway, NJ, USA). Anti-mouse antibodies against CCR5, CCR7, CXCR3, and CXCR4 were purchased from Cell Signaling Technology (Danvers, MA, USA).

### Immunohistochemistry/migration assay

MSCs were intravenously injected into 20-week-old MRL.Fas^{lpr} mice (1 x 10⁶ cells/injection). After 24 hours, the kidneys were isolated from the MRL.Fas^{lpr} mice, fixed with 4% formalin, and immersed in PBS. After dehydration with ethanol and xylene, the tissue was inserted into paraffin and cut into 4 µm sections, and after the sections were heated in a microwave oven (650 W, 20 minutes) for antigen retrieval, endogenous peroxidase activity was blocked with 3% hydrogen peroxide. Thereafter, the sections were incubated with a primary antibody (mouse antibody against mouse MHC-1 H-2Db (diluted 1:100; Abcam, Cambridge, UK), overnight incubation at 4°C). Thereafter, all the sections were incubated with a secondary antibody anti-mouse IgG conjugated with horseradish peroxidase (HRP; Vector Laboratories, Burlingame, CA, USA) at room temperature for 1 hour. Signals were developed using a two-component high-sensitivity diaminobenzidine (DAB) chromogenic substrate for 10 hours, and counter-staining was performed with hematoxylin.

### Cell binding assay

Endothelial cells (1 × 10⁶ cells/ml) were labeled with MSCs containing 0.5 µM CMFDA (Life Technologies) and 5 µM CMTPX (Thermo Fisher Scientific) in a serum-free medium at 37°C for 10 minutes. After staining, the cells were washed twice in a culture medium containing 10% FBS. After MSCs (1 × 10⁶) and endothelial cells (1 × 10⁶) were mixed in a 12 × 75 mm polystyrene tube (BD Biosciences) and centrifuged at 800 rpm for 1 minute, a pellet was cultured at 37°C for 10 minutes. Subsequently, a cell mixture was gently suspended and analyzed by flow cytometry. A conjugation ratio was calculated as a portion of the CMFDAC MTPX double-positive events.

### Statistical analysis

The data is expressed as the mean ± SEM of 3 or more independent experiments performed *in vitro* or *in vivo* (6 mice). The p-value was calculated using one-way ANOVA from GraphPad Prism Software (San Diego, CA, USA).

### Experimental results

### Therapeutic activity analysis of CXCR3 -/- MSCs

First, the therapeutic activity of CXCR3 -/- MSC (CXCR3-deficient MSC) was confirmed in the MRL.Fas^{lpr} mouse model. The wild-type (WT) MSCs remarkably prolonged the mouse survival period: 66% of rats that received WT MSCs survived up to 22 weeks, whereas only 16% of control rats survived (FIG. 1A). Meanwhile, only 33% of rats that received CXCR3 -/- MSCs survived (FIG. 1A). It was confirmed that MSCs had no effect on body weight (FIG. 1B). The WT MSCs reduced serum and proteinuria levels (FIG. IE) of anti-dsDNA (FIG. 1C) and total IgG antibodies (FIG. 1D), but the CXCR3 -/- MSCs did not. Cyclophosphamide used as a positive control also alleviated the disease. This data shows that CXCR3 - /- MSCs cannot ameliorate lupus symptoms in the MRL.Fas^{lpr} mouse.

### Renal infiltration analysis of MSCs

FIG. 2 is an analysis of renal infiltration effects of mesenchymal stem cells expressing CXCR3. It was confirmed that WT MSCs migrated (homing) to nephritic kidney better than CXCR3 -/- MSC cells (FIG. 2A). The kidneys of 22-week-old MRL.Fas^{lpr} mice expressed higher levels of CXCL10 than 8-week-old mice, but both mice expressed CXCL12 similarly (FIG. 2B). This data suggests that the nephritic kidney may express high levels of CXCL10, which may increase MSC induction to the kidneys. Next, it was analyzed how CXCL10 increased MSC infiltration. CXCL10 increased conjugation of WT MSCs with endothelial cells, which is an important step for infiltration of MSCs into the kidneys. However, CXCR3 -/- MSCs did not increase conjugation with endothelial cells (FIG. 2C). CXCL10 also increased the expression of MMP-2 and MMP-9, which are important enzymes for interstitial migration) (FIG. 2D), but did not affect the expression of α4/β1 integrin and CD44 by MSCs (FIG. 2E). These results suggest that CXCL10 is an important chemokine that enhances MSC infiltration into the nephritic kidney.

### Immunosuppressive capacity analysis of CXCR3 -/- MSCs

Despite the defect in the renal infiltration capacity of CXCR3 -/- MSCs, a phenotype similar to WT MSCs was exhibited. CD34 and MHC-II known as a marker of MSCs were expressed, but CD73 and Sca-1 were not expressed. As a result of analysis using RT-PCR, real-time PCR, and western blot (WB), it was confirmed that WT MSCs expressed CCR5, CXCR3, and CXCR4, but CXCR3 -/-MSCs did not express CXCR3, and expressed CCR5 and CXCR4 (FIG. 3A). Further, CXCR3 -/- MSCs did not migrate towards CXCL10 in a Transwell assay (FIG. 3B). However, CXCR3 -/- MSCs suppressed T cells and B cells from producing IFN-γ and IgM, respectively (FIG. 3C, FIG. 3D), like WT MSCs. Like WT MSCs, CXCR3 -/- MSCs expressed iNOS, TGF-β, and COX-2 mRNA (FIG. 3E), and produced NO, TGF-β, and PGE₂ (FIG. 3F). These results suggest that CXCR3 -/- MSCs have a phenotype and immunosuppressive capacity similar to those of WT MSCs, except that CXCR3 -/- MSCs cannot migrate to CXCL10.

The renal infiltration of MSCs is a multi-step process: The first step is systemic administration of MSCs, which are delivered to the inflammatory site through the vascular system. The second step includes capture of MSCs in blood, rolling, adhesion to the endothelium, and transendothelial migration. The last step is interstitial migration. The rolling and adhesion of leukocytes depend on L-selectin, VLA-4, and LFA-1, but this might not be the case for MSCs. It has been reported that endothelial cells of inflammatory tissue express E-selectin, P-selectin, VCAM-1, and ICAM-1, MSCs express CD44 and α4β1 integrin, and by interaction thereof, MSCs can be firmly attached to endothelial cells. According to our data, MSCs express CD44 and VLA-4 well even in the absence of CXCL10 stimulation, which may mean that MSCs binding to endothelial cells independently act on CXCL10. As another CXCL10-independent mechanism, MSCs larger than leukocytes may be non-specifically detained in microcapillaries. Further studies are required to clarify how CXCL10 increases the conjugation of MSCs and endothelial cells.

The nephritic kidneys of lupus-prone mice and patients with SLE express high levels of CCL2, CCL3, CCL5, CXCL10, CXCL12, CXCL13, and CX3CL1, which induces the migration of inflammatory cells to the nephtritic kidneys. Patients with SLE have more CXCR3 + T cells in their kidneys and less CXCR3 + T cells in their blood. CXCL10 is most abundant in patients with SLE and is distributed in the same areas as CXCR3 + immune cells in the kidney. Immune cell infiltration into the nephritic kidney is well known, but little is known about MSC infiltration. MSCs are known to express CCR2, CCR5, CXCR3, and CXCR4. Among them, CCR2 and CXCR4 are well-known as regulators for MSC infiltration into the heart and skin. However, it is not known whether CXCR3 is required for MSCs to migrate to the nephritic kidney. The present experimental data shows that CXCR3 plays an important role in MSC infiltration into the nephritic kidney. It was confirmed that CXCR3-deficient MSCs have less binding strength to endothelial cells, less MMP9 expression, less infiltration into the nephritic kidney in MRL.Fas^{lpr} mice than WT MSCs, resulting in low improvement effects of lupus symptoms. However, CXCR3-deficient MSCs showed proliferation and immunosuppressive capacities similar to WT MSCs.

The present invention may affect the development of non-invasive biomarkers for diagnostic classification and progression lupus nephritis. Urinary CXCL10 is reduced with remission to inactive lupus nephritis (LN) in patients with SLE, suggesting that urinary CXCL10 may be a good biomarker for monitoring LN improvement in patients with SLE after treatment. Genetic manipulation of MSCs with respect to CXCR3 may be a promising strategy to improve therapeutic efficacy in patients with SLE. Understanding the process of MSC infiltration into the damaged site can provide a tool to improve the therapeutic efficacy of MSCs.

### [Industrial Applicability]

According to the present invention, it is possible to provide a biomarker for predicting effectiveness for autoimmune disease treatment, a diagnostic kit, and a use for treatment. In particular, the present invention can provide a biomarker for predicting the effectiveness of mesenchymal stem cells (MSCs) comprising the chemokine receptor CXCR3 for autoimmune disease treatment; a biomarker comprising the chemokine CXCL10 for predicting the prognosis of autoimmune disease treatment; a diagnostic kit comprising an agent capable of measuring the expression of the biomarker; and a pharmaceutical composition for prevention or treatment of an autoimmune disease, which comprises mesenchymal stem cells having an upregulated expression level of the chemokine receptor CXCR3. In particular, the present invention can clearly elucidate the role of the CXCL10-CXCR3 axis in MSC infiltration, and thus can show or improve a preventive or therapeutic effect on an autoimmune disease, particularly systemic lupus erythematosus.

## Claims

1. A biomarker composition for predicting effectiveness of mesenchymal stem cells comprising a chemokine receptor CXCR3 for autoimmune disease treatment.

2. The biomarker composition of claim 1, wherein the autoimmune disease is systemic lupus erythematosus or complications caused thereby.

3. A kit comprising an agent which measures an expression level of CXCR3 or an expression level of mRNA thereof for predicting effectiveness of mesenchymal stem cells for autoimmune disease treatment.

4. The kit of claim 3, wherein an agent which measures the expression level of CXCR3 is an antibody specifically binding to CXCR3, and an agent which measures the expression level of mRNA of CXCR is a primer, probe or antisense nucleotide specifically binding to CXCR3.

5. The kit of claim 3, wherein when CXCR3 or mRNA thereof is not expressed, or is expressed at a level lower than that of wild-type mesenchymal stem cells, the therapeutic effectiveness is absent or low.

6. A biomarker composition comprising a chemokine CXCL10 for predicting prognosis of autoimmune disease treatment, wherein the autoimmune disease treatment is a treatment using mesenchymal stem cells comprising CXCR3.

7. The biomarker composition of claim 6, wherein the CXCL10 is expressed in the kidneys.

8. The biomarker composition of claim 7, wherein in the kidneys expressing CXCL10, CXCL10 induces mesenchymal stem cells comprising CXCR3 to infiltrate into the kidneys.

9. The biomarker composition of any one of claims 6 to 8, wherein the autoimmune disease is systemic lupus erythematosus or complications caused thereby.

10. A method for providing information for predicting effectiveness of mesenchymal stem cells for autoimmune disease treatment by measuring an expression level of a chemokine receptor CXCR3 expressed in mesenchymal stem cells (MSCs).

11. A method for screening mesenchymal stem cells exhibiting an effective therapeutic effect on an autoimmune disease by measuring an expression level of a chemokine receptor CXCR3.

12. A pharmaceutical composition comprising the mesenchymal stem cells screened according to claim 11 for preventing or treating an autoimmune disease.

13. A pharmaceutical composition for preventing or treating an autoimmune disease, which comprises: a chemokine receptor CXCR3 of mesenchymal stem cells; or an agent which increases the expression level of CXCR3.

14. The pharmaceutical composition of claim 13, wherein the agent which increases the expression level of CXCR3 specifically binds to CXCR3.

15. A pharmaceutical composition for preventing or treating an autoimmune disease, comprising, as an active ingredient, mesenchymal stem cells having an upregulated expression level of a chemokine receptor CXCR3 compared to the wild type.

16. The pharmaceutical composition of claim 15, wherein the mesenchymal stem cells having an upregulated expression level of CXCR3 compared to the wild type have: (i) positive immunological properties for at least one surface antigen selected from the group consisting of CD105, CD29, CD44, CD73, and CD90; and (ii) negative immunological properties for at least one surface antigen selected from the group consisting of CD34, CD45, and HLA-DR.

17. The pharmaceutical composition of claim 15, wherein the autoimmune disease is selected from the group consisting of lupus (systemic lupus erythematosus), rheumatoid arthritis, progressive systemic sclerosis (scleroderma), atopic dermatitis, alopecia areata, psoriasis, pemphigus, asthma, aphthous stomatitis, chronic thyroiditis, inflammatory enteritis, Behcet's disease, Crohn's disease, dermatomyositis, polymyositis, multiple sclerosis, autoimmune hemolytic anemia, autoimmune encephalomyelitis, myasthenia gravis, Grave's disease, polyarteritis nodosa, ankylosing spondylitis, fibromyalgia syndrome, and temporal arteritis.

18. The pharmaceutical composition of claim 15, wherein the autoimmune disease is systemic lupus erythematosus.

19. A method for preventing or treating an autoimmune disease, the method comprising administering, to an individual, or the individual taking, a pharmaceutical composition comprising the mesenchymal stem cells screened according to claim 11.

20. A use of a pharmaceutical composition comprising the mesenchymal stem cells screened according to claim 11 for preventing or treating an autoimmune disease.

21. A method for preventing or treating an autoimmune disease, the method comprising administering, to an individual, or the individual taking, a pharmaceutical composition comprising: a chemokine receptor CXCR3 of mesenchymal stem cells; or an agent which increases an expression level of CXCR3.

22. A use of a pharmaceutical composition comprising: a chemokine receptor CXCR3 of mesenchymal stem cells; or an agent which increases an expression level of CXCR3 for preventing or treating an autoimmune disease.

23. A method for preventing or treating an autoimmune disease, the method comprising administering, to an individual, or the individual taking, a pharmaceutical composition comprising, as an active ingredient, mesenchymal stem cells having an upregulated expression level of a chemokine receptor CXCR3 compared to the wild type.

24. A use of a pharmaceutical composition comprising, as an active ingredient, mesenchymal stem cells having an upregulated expression level of a chemokine receptor CXCR3 compared to the wild type for preventing or treating an autoimmune disease.
